# EUROPEAN PATENT APPLICATION

(11) **EP 4 628 033 A1**
(43) Date of publication of application: **08.10.2025**
(21) Application number: 23896903.4
(22) Date of filing: 30.11.2023
(51) Int. Cl.: A61B 17/22

(54) **LUMEN OPENING INSTRUMENT, LUMEN OPENING SYSTEM, AND LUMEN OPENING METHOD**

(30) Priority: 02.12.2022 CN 202211533090
(71) Applicant: Ultratellege Co., Ltd., Beijing 102628 (CN)
(72) Inventor: LIU, Ying, Beijing 102628 (CN); SONG, Tao, Beijing 102628 (CN); DU, Shu, Beijing 102628 (CN); DONG, Yonghe, Beijing 102628 (CN); ZHU, Mingzhe, Beijing 102628 (CN); YANG, Zheng, Beijing 102628 (CN)
(74) Representative: Kransell & Wennborg KB
(86) International application number: PCT/CN2023/135636
(87) International publication number: WO 2024/114761

(57) **Abstract**

A lumen opening instrument, a lumen opening system, and a lumen opening method. The lumen opening instrument comprises an inner layer tube assembly (100) and an outer layer tube assembly (200); the inner layer tube assembly (100) comprises an inner tube (1) and an ultrasonic connector assembly (2) connected to the proximal end of the inner tube (1); the outer layer tube assembly (200) comprises an outer tube (3) and at least one expansion and contraction portion (4) connected to the distal end of the outer tube (3); the outer tube (3) and said expansion and contraction portions (4) are all sleeved on the outside of the inner tube (1); and the outer layer tube assembly (200) can move relative to the inner tube (1), so as to cause the expansion and contraction portions (4) to transition between an expanded state and a contracted state.

## Description

### CROSS-REFERENCE TO RELATED APPLICATION

The present disclosure claims the priority to the Chinese patent application with the filling No. 2022115330901 filed with the Chinese Patent Office on December 2, 2022, and entitled "LUMEN OPENING INSTRUMENT, AND LUMEN OPENING SYSTEM", the contents of which are incorporated herein by reference in entirety.

### TECHNICAL FIELD

The present disclosure relates to the technical field of medical devices, and in particular, to a lumen recanalization device (lumen opening instrument), a lumen recanalization system (lumen opening system), and a lumen recanalization method (lumen opening method).

### BACKGROUND ART

Endovascular interventional therapy becomes a preferred treatment method for many diseases currently due to its minimally invasive nature. Commonly used endovascular interventional instruments include guidewires, balloons, stents, debulking devices, etc. In the prior art, a treatment process at least includes as follows. Firstly, a guidewire is pushed through a puncture site along a human lumen to a lesion site. To avoid damaging the lumen during the pushing process, a head end of a pilot guidewire is generally soft, so that the guidewire cannot directly pass through the lesion after reaching the lesion site, and thus it is necessary to insert a catheter and exchange for a guidewire with good passability through the catheter to pass through the lesion. For some occlusive lesions, an occluded segment is accompanied by a hard fibrous cap and calcified plaques, with a hard texture, resulting in problems such as long guidewire passage time, vascular wall injury, and low recanalization success rate. Secondly, a balloon catheter is pushed along the guidewire to further expand a size of an inner lumen of the occluded segment. Since the balloon cannot achieve true debulking, and lumen recoil results in poor dilation effect. Thirdly, when the effect is poor, a stent needs to be implanted to dilate the lumen, but stent implantation stimulates endothelial hyperplasia, resulting in in-stent restenosis and a very high medium-to-long term occlusion rate. The entire process above requires the alternating use of recanalization and expansion devices, resulting in cumbersome operation and long operation time. Recanalization failure, poor dilation effect, and stent restenosis are urgent problems to be solved in current endovascular interventional therapy procedures.

In addition, existing technologies that need to be understood further include as follows. A known ultrasonic energy transmission system includes a generator, a transducer assembly (ultrasonic handle), and a catheter or guidewire. The generator is configured to generate, control, amplify, and/or transmit an alternating electronic signal (e.g., voltage signal) of a desired frequency to the transducer assembly. The transducer assembly (ultrasonic handle) typically includes one or more piezoelectric ceramics, which expand and contract at high frequencies when excited by high-frequency electronic signals. These high-frequency vibrations are amplified into ultrasonic energy by a horn, and the ultrasonic energy is transmitted to a catheter or guidewire. The ultrasonic energy is transmitted to a distal end of the catheter or guidewire to ablate and/or otherwise destroy body lesions.

### SUMMARY

The purpose of the present disclosure is to provide a lumen recanalization device, a lumen recanalization system, and a lumen recanalization method, which alleviate the problems in existing endovascular interventional therapy procedures for occlusive lesions, such as the difficulty of the device in passing through the occluded segment of the lesion, the cumbersome operation caused by the alternating use of recanalization and expansion devices, and the low long-term patency rate caused by the in-stent restenosis due to stent implantation required by poor debulking effect of balloon dilation, thereby improving an immediate success rate and a medium-to-long term effects of a procedure.

To achieve the above purpose, the embodiments of the present disclosure adopt the following technical solutions.

In a first aspect, a lumen recanalization device is provided in embodiments of the present disclosure. The lumen recanalization device comprises an inner tube assembly and an outer tube assembly. Specifically, the inner tube assembly comprises an inner tube and an ultrasonic connector assembly connected to a proximal end of the inner tube. The outer tube assembly comprises an outer tube and at least one expansion-contraction portion connected to a distal end of the outer tube. Both the outer tube and the at least one expansion-contraction portion are sleeved outside the inner tube. An expansion-contraction portion is configured to have a contracted state where it integrally adheres to or fits closely to an outer wall of the inner tube, and an expanded state where it bulges outward from the inner tube along a radial direction of the inner tube. The outer tube assembly is capable of moving relative to the inner tube to enable the expansion-contraction portion to transition between the expanded state and the contracted state.

A usage method and at least achievable functional effects of the lumen recanalization device provided in the embodiments are as follows.

An ultrasonic device is required during use. The ultrasonic device generally comprises an ultrasonic generator and an ultrasonic transducer connected to each other. Some ultrasonic devices further comprise a foot-driven pedal. The ultrasonic generator may be driven by the foot-driven pedal to start the ultrasonic device in cooperation. There are also ultrasonic devices with other driving methods, which all belong to the prior art and will not be repeated in the present disclosure.

The ultrasonic connector assembly at the proximal end of the inner tube is connected to the ultrasonic transducer of the ultrasonic device, and each expansion-contraction portion is ensured to be in the contracted state, and then a distal end of the inner tube of the lumen recanalization device is extended into an occluded site of a lesion.

Then, in a first step, the ultrasonic device is started to recanalize the occluded site with the help of ultrasonic energy. This recanalization method mainly applies a combined mechanism of mechanical vibration and cavitation, and uses ultrasonic energy for lumen recanalization. Mechanical vibrations at an ultrasonic frequency reach a lesion location along the recanalization device. When the device vibrates at a high frequency, the cavitation occurs, and tiny bubbles are generated around the lesion. These tiny bubbles oscillate in alternating periodic vibration waves, and the bubbles are alternately stretched and compressed. At a moment when the bubbles are compressed to collapse, an instantaneous pressure is generated, and this instantaneous pressure jump forms a shock wave. The explosion of countless bubbles is like countless micro-bombs, causing damage to an internal structure of an occluded segment, thereby allowing the device to quickly pass through the lesion location while expanding the lumen. More specifically, the high-frequency and low-amplitude mechanical vibrations generated by ultrasound may cause the distal end of the inner tube to penetrate the hard fibrous cap like a vibrating "hand-held hammer". The high-frequency vibrations of the inner tube generate strong negative and positive pressure cycles in the surrounding fluid. When the distal end of the inner tube retracts, dissolved gases on a negative side of a pressure cycle form cavitation bubbles, which rapidly collapse on a positive side of the pressure cycle. The collapse of cavitation bubbles produces strong mechanical shock waves that erode calcified plaques. Moreover, therapeutic ultrasound for the recanalization of the occluded segment also has the characteristic of selective plaque ablation. Tissues with high collagen and elastin content show strong resistance to ultrasonic damage, while tissues lacking these components are easily destroyed. High-intensity ultrasonic waves disrupt fibrous or calcified plaques without damaging normal arterial vessels. This recanalization method of the occluded segment is more efficient, which is conducive to greatly shortening the recanalization time of the occluded segment during the procedure, and avoiding the problem that the guidewire cannot pass through due to an excessive hardness of the occluded segment, thereby improving the recanalization success rate of the occluded segment.

In a second step, the outer tube assembly is operated to move the outer tube assembly relative to the inner tube, so that each expansion-contraction portion transitions from the contracted state to the expanded state, thereby expanding a hole formed by recanalization in the first step to further expand a size of an inner lumen of the occluded segment while supporting the inner tube to indirectly improve a supporting force for supporting the guidewire extended into the inner tube. Under an action of this strong supporting force, the guidewire may have a strong directionality and is not easy to bend, thereby further shortening recanalization time of the occluded segment and improving recanalization efficiency of the occluded segment.

In particular, the first step and the second step described above may also be carried out in a reverse order. That is, the outer tube assembly is first operated to move the outer tube assembly relative to the inner tube, so that each expansion-contraction portion transitions from the contracted state to the expanded state. The expansion-contraction portion in the expanded state supports the inner tube. Then, the ultrasonic device is started to recanalize the occluded site with the help of ultrasonic energy. Preferably, the first step and the second step are alternatively performed according to the needs of an operator.

In summary, the lumen recanalization device provided in the embodiments alleviates the problem that the device is difficult to pass through the occluded segment of the lesion in existing endovascular interventional therapy procedures for occlusive lesions, and achieves good effects of shortening the recanalization time of the occluded segment and improving the recanalization efficiency of the occluded segment. Moreover, as analyzed above, surgical instruments are not replaced during the entire process of recanalizing and dilating the occluded segment of the lesion, thereby alleviating the problem of cumbersome operation caused by the alternating use of the recanalization and expansion devices in existing endovascular interventional therapy procedures for occlusive lesions, and alleviating the problem of low long-term patency rate caused by the in-stent restenosis due to the stent implantation required by poor debulking effect of balloon dilation. Compared with the prior art, the lumen recanalization device provided in the embodiments is simpler and more convenient to use, and the surgical process is faster and more convenient, which is conducive to further improving the surgical efficiency, reducing the surgical risk of various complications caused by excessive operation time, and further improving an immediate success rate and medium-to-long effects of a procedure (It should be noted that the effects achievable by the lumen recanalization device provided in the present disclosure are only relative to the lumen recanalization devices provided in the prior art and most patient conditions, and due to numerous influencing factors in an actual surgical treatment, it does not mean that the use of the lumen recanalization device provided in the present disclosure can guarantee the absolute safety and effectiveness of treatment for any patient with a related disease).

In some preferred implementations of the embodiments of the present disclosure, a distal end of the inner tube extends beyond a portion of the at least one expansion-contraction portion most proximate to the distal end of the inner tube. In a further preferred implementation, a distal end of the inner tube extends beyond a portion of the at least one expansion-contraction portion most proximate to the distal end of the inner tube.

An expansion-contraction portion most proximate to a distal end of the inner tube is a distal expansion-contraction portion. In some optional implementations of the embodiments of the present disclosure, the expansion-contraction portion(s) only comprise the distal expansion-contraction portion, that is, there is one and only one expansion-contraction portion. In some other optional implementations of the embodiments of the present disclosure, at least two expansion-contraction portions exist, and an expansion-contraction portion farthest from the distal end of the inner tube is a proximal expansion-contraction portion. The at least two expansion-contraction portions are sequentially connected end to end, and the distal end of the outer tube is connected to a proximal end of the proximal expansion-contraction portion; or the outer tube comprises a proximal tube and a plurality of distal tubes, the at least two expansion-contraction portions are spaced from each other, two adjacent expansion-contraction portions are connected through at least one distal tube, and a distal end of the proximal tube is connected to the proximal end of the proximal expansion-contraction portion.

In some optional implementations of the embodiments of the present disclosure, a portion of the at least one expansion-contraction portion most proximate to a distal end of the inner tube is fixed to an outer wall of a distal end of the inner tube. In these optional implementations, further optionally, the outer tube is capable of rotating relative to the inner tube to enable the expansion-contraction portion to transition between the expanded state and the contracted state; or, further optionally, the outer tube is capable of sliding back and forth relative to the inner tube to enable the expansion-contraction portion to transition between the expanded state and the contracted state.

In some other optional implementations of the embodiments of the present disclosure, an outer wall of a distal end of the inner tube is provided with a limiting structure, and a portion of the at least one expansion-contraction portion most proximate to the distal end of the inner tube is limited to the outer wall of the distal end of the inner tube by the limiting structure.

Preferably, a distal end of the distal expansion-contraction portion is further connected to a constraint fixation component. The constraint fixation component is used as a portion fixedly connected to the outer wall of the distal end of the inner tube in the above some implementations, or as a portion limited by the limiting component in the above other implementations. Further optionally, the constraint fixation component comprises a fixing ring or a fixing tube, and the fixing ring or the fixing tube is sleeved outside the inner tube.

In some optional implementations, the outer wall of the distal end of the inner tube is provided with the above limiting structure. The distal end of the distal expansion-contraction portion is connected to the above constraint fixation component, and the constraint fixation component is limited to the outer wall of the distal end of the inner tube by the limiting structure. The outer tube is capable of sliding back and forth relative to the inner tube to enable the expansion-contraction portion to transition between the expanded state and the contracted state. In this case, the limiting structure on the outer wall of the distal end of the inner tube described above comprises a distal blocking protrusion, and the constraint fixation component of the distal expansion-contraction portion is blocked on a proximal side of the distal blocking protrusion. Preferably, the limiting structure further comprises a proximal blocking protrusion, but it is not limited thereto. The proximal blocking protrusion is located on a proximal side of the constraint fixation component of the distal expansion-contraction portion.

In some optional implementations of the embodiments of the present disclosure, the outer tube assembly further comprises an outer sheath tube, and the outer sheath tube is sleeved outside the outer tube. The outer sheath tube is capable of sliding back and forth relative to the outer tube, so as to retract or release the expansion-contraction portion in a gap between an inner wall of the outer sheath tube and an outer wall of the outer tube.

In optional implementations of the embodiments of the present disclosure, preferably, the expansion-contraction portion comprises a framework, but it is not limited thereto. The framework is in a mesh shape or is formed by a plurality of connection wires extending along the axial direction of the inner tube and arranged at intervals. Further preferably, the expansion-contraction portion further comprises a membrane connected to the framework, but it is not limited thereto. The membrane is made of a flexible material. In some optional implementations of the embodiments of the present disclosure, the outer tube assembly further comprises a sealing device, and the sealing device is configured to seal a tube gap between an inner wall of a proximal end of the outer tube and the outer wall of the inner tube along the radial direction.

Further optionally, the outer tube assembly further comprises a sliding handle. The sliding handle is connected to the proximal end of the outer tube and sleeved outside the inner tube, and the sliding handle is configured to drive the outer tube to slide back and forth relative to the inner tube.

Optionally, the sealing device is arranged between an inner wall of the sliding handle and the outer wall of the inner tube to seal the gap between the inner wall of the sliding handle and the outer wall of the inner tube along the radial direction, thereby indirectly sealing the gap between the inner wall of the proximal end of the outer tube and the outer wall of the inner tube along the radial direction.

In an optional implementation, a handle liquid passage hole is provided on the sliding handle. The handle liquid passage hole is communicated with the gap between the outer tube and the inner tube, and the handle liquid passage hole is located on a distal side of the sealing device.

In another optional implementation, the sealing device is arranged in the tube gap between the inner wall of the proximal end of the outer tube and the outer wall of the inner tube, thereby directly sealing the gap between the inner wall of the proximal end of the outer tube and the outer wall of the inner tube along the radial direction.

Optionally, a sliding position mark is provided on the sliding handle, and scale lines arranged along the axial direction of the inner tube are provided on an outer wall of the proximal end of the inner tube. The sliding position mark and a scale line cooperate with each other to display a distance that the outer tube slides back and forth relative to the inner tube.

In optional implementations of the embodiments of the present disclosure, an outer tube liquid passage hole penetrating a tube wall of the outer tube is provided on a wall of a proximal end of the outer tube.

In optional implementations of the embodiments of the present disclosure, an inner tube liquid passage hole penetrating a tube wall of the inner tube is provided on a wall of the proximal end of the inner tube.

In any of the above optional implementations of the embodiments, preferably, the ultrasonic connector assembly comprises an ultrasonic connector. A proximal end of the ultrasonic connector is provided with a connection portion configured to be connected to an ultrasonic transducer. The ultrasonic connector is provided with an inner tube connection hole and a connector liquid passage hole. The proximal end of the inner tube is connected to a distal end of the inner tube connection hole, and a lumen of the inner tube is communicated with the inner tube connection hole. The connector liquid passage hole is communicated with the inner tube connection hole.

Further preferably, the ultrasonic connector assembly further comprises a hose and a check valve assembly. An end of the hose is connected to the connector liquid passage hole. The check valve assembly that only allows a liquid to flow from the hose into the inner tube connection hole through the connector liquid passage hole is connected to a portion of the hose away from the connector liquid passage hole.

In a second aspect, a lumen recanalization system is provided in embodiments of the present disclosure. The lumen recanalization system comprises an ultrasonic generator, an ultrasonic transducer, and the lumen recanalization device in any of the above implementations. the ultrasonic transducer is connected to the ultrasonic generator, and the ultrasonic connector assembly is connected to the ultrasonic transducer.

In a third aspect, a lumen recanalization method is provided in embodiments of the present disclosure. The lumen recanalization method comprises using the above lumen recanalization system in the second aspect to feed a distal end of the lumen recanalization device into an occluded site in a vascular lumen of a patient, and starting the ultrasonic generator to drive the inner tube to vibrate. While the inner tube vibrates ultrasonically, ultrasonic energy of the inner tube is capable of being transmitted to the expansion-contraction portion connected to the distal end of the outer tube, so that the expansion-contraction portion in the expanded state cuts thrombus.

Since the lumen recanalization method provided in the embodiments of the present disclosure uses the above lumen recanalization system, which in turn comprises the above lumen recanalization device, the lumen recanalization method and the lumen recanalization system provided in the embodiments of the present disclosure are capable of achieving all the beneficial effects achievable by the above lumen recanalization device.

### BRIEF DESCRIPTION OF DRAWINGS

To more clearly illustrate the specific embodiments of the present disclosure or the technical solutions in the prior art, the drawings required for describing the specific embodiments or the prior art will be briefly introduced below. Obviously, the drawings described below are some embodiments of the present disclosure. Other drawings can also be obtained by those of ordinary skill in the art without creative efforts based on these drawings.
FIG. 1 is a schematic diagram of an overall structure of a lumen recanalization device provided in embodiments of the present disclosure when an expansion-contraction portion is in an expanded state;
FIG. 2 is an enlarged view of a partial structure of part A in FIG. 1;
FIG. 3 is a schematic diagram of an overall structure of a lumen recanalization device provided in embodiments of the present disclosure when an expansion-contraction portion is in a contracted state;
FIG. 4 is an enlarged view of a partial structure of part A' in FIG. 3;
FIG. 5 is a schematic diagram of a first optional structure of an expansion-contraction portion in a lumen recanalization device provided in embodiments of the present disclosure;
FIG. 6 is a schematic diagram of a second optional structure of an expansion-contraction portion in a lumen recanalization device provided in embodiments of the present disclosure;
FIG. 7 is a schematic diagram of a third optional structure of an expansion-contraction portion in a lumen recanalization device provided in embodiments of the present disclosure;
FIG. 8 is a schematic diagram of a fourth optional structure of an expansion-contraction portion in a lumen recanalization device provided in embodiments of the present disclosure;
FIG. 9 is a schematic diagram of a connection structure in an expanded state between a distal end of a distal expansion-contraction portion and an inner tube in a lumen recanalization device provided in embodiments of the present disclosure;
FIG. 10 is a schematic diagram of the connection structure in a contracted state between the distal end of the distal expansion-contraction portion and the inner tube shown in FIG. 9;
FIG. 11 is a schematic diagram of another connection structure in an expanded state between a distal end of a distal expansion-contraction portion and an inner tube in a lumen recanalization device provided in embodiments of the present disclosure;
FIG. 12 is a schematic diagram of the connection structure in a contracted state between the distal end of the distal expansion-contraction portion and the inner tube shown in FIG. 11;
FIG. 13 is a sectional view of an overall structure of a sliding handle in some optional embodiments of a lumen recanalization device provided in embodiments of the present disclosure;
FIG. 14 is a sectional view of an overall structure of an ultrasonic connector in some optional embodiments of a lumen recanalization device provided in embodiments of the present disclosure;
FIG. 15 is a schematic diagram of an overall structure of a lumen recanalization system provided in embodiments of the present disclosure;
FIG. 16 is a schematic diagram of a distal end of a catheter in a recanalization stage when a surgical treatment is performed using a lumen recanalization device or a lumen recanalization system provided in embodiments of the present disclosure;
FIG. 17 is a schematic diagram of an expansion-contraction segment in a stage entering an occluded segment when a surgical treatment is performed using a lumen recanalization device or a lumen recanalization system provided in embodiments of the present disclosure;
FIG. 18 is a schematic diagram of an expansion-contraction segment in an expansion stage when a surgical treatment is performed using a lumen recanalization device or a lumen recanalization system provided in embodiments of the present disclosure;
FIG. 19 is a schematic diagram of an assembly structure of distal ends of an outer sheath tube, an outer tube, and an inner tube in a lumen recanalization device provided in embodiments of the present disclosure when the outer sheath tube is included in an outer tube assembly;
FIG. 20 is a schematic diagram of a matching structure between a sliding position mark on a sliding handle and a scale line on an inner tube in a lumen recanalization device provided in embodiments of the present disclosure;
FIG. 21 is a schematic diagram of a tip structure of a distal end of an inner tube in some embodiments;
FIG. 22 is a partial schematic diagram of a tube structure in some embodiments, in which a proximal end of an outer tube is provided with an outer tube liquid passage hole and a proximal end of an inner tube is provided with an inner tube liquid passage hole.

Reference numerals: 1-inner tube; 101-tip; 102-scale line; 103-inner tube liquid passage hole; 11-limiting structure; 111-distal blocking protrusion; 112-proximal blocking protrusion; 2-ultrasonic connector assembly; 21-ultrasonic connector; 210-connection portion; 211-inner tube connection hole; 212-connector liquid passage hole; 22-hose; 23-check valve assembly; 3-outer tube; 31-liquid spraying hole; 32-outer tube liquid passage hole; 4-expansion-contraction portion; 41-framework; 42-constraint fixation component; 5-sliding handle; 501-sliding position mark; 51-handle liquid passage hole; 52-sealing device; 6-ultrasonic generator; 7-ultrasonic transducer; 8-foot-driven pedal; 9-outer sheath tube.

### DETAILED DESCRIPTION OF EMBODIMENTS

To make the objectives, technical solutions, and advantages of the embodiments of the present disclosure clearer, the technical solutions in the embodiments of the present disclosure will be described clearly and completely below in combination with the drawings in the embodiments of the present disclosure. Obviously, the described embodiments are part of the embodiments of the present disclosure, not all of them. Usually, the components of the embodiments of the present disclosure described and shown in the drawings herein can be arranged and designed in various different configurations.

Therefore, the following detailed description of the embodiments of the present disclosure provided in the drawings is not intended to limit the scope of the claimed present disclosure, but only represents the selected embodiments of the present disclosure. Based on the embodiments in the present disclosure, all other embodiments obtained by those of ordinary skill in the art without creative efforts fall in the protection scope of the present disclosure.

It should be noted that similar reference numerals and letters represent similar items in the following drawings. Therefore, once an item is defined in one drawing, it does not need to be further defined and explained in subsequent drawings.

In the description of the present disclosure, it should be noted that an orientation or positional relationship indicated by the term such as "upper", "lower", "vertical", "horizontal", "inner", "outer" is based on an orientation or positional relationship shown in the drawings, or an orientation or positional relationship that a product of the present disclosure is usually placed in use, which is only for the convenience of describing the present disclosure and simplifying the description, rather than indicating or implying that the referred device or element must have a specific orientation, be constructed and operated in a specific orientation, and thus cannot be understood as a limitation to the present disclosure. In addition, the term such as "first", "second", "third" is only configured to distinguish the description, and cannot be understood as indicating or implying relative importance.

In the description of the present disclosure, it should also be noted that unless otherwise clearly specified and limited, the terms such as "arrange", "install", and "connect" should be interpreted broadly. For example, "connection" may be a fixed connection, a detachable connection, or an integral connection; may be a mechanical connection, or an electrical connection; may be a direct connection, or an indirect connection through an intermediate medium; may be an internal communication between two elements. For those of ordinary skill in the art, the specific meanings of the above terms in the present disclosure may be understood according to specific situations.

Particularly, in the present disclosure, during a procedure, an end of a medical device proximate to an operator is a proximal end of the medical device, and an end of the medical device entering a patient's blood vessel is a distal end of the medical device (a front end of the medical device is the distal end, and a rear end of the medical device is the proximal end). An axial direction refers to a direction parallel or substantially parallel to a line connecting a center of the distal end and a center of the proximal end of the medical device. A radial direction refers to a direction perpendicular or substantially perpendicular to the above axial direction.

Some embodiments of the present disclosure are described in detail below in combination with the drawings. The following embodiments and the features in the embodiments may be combined with each other without conflict.

Endovascular interventional therapy is currently a preferred treatment method for occlusive lesions caused by atherosclerosis. Commonly used endovascular interventional instruments include guidewires, balloons, stents, debulking devices, etc. In the prior art, a treatment process at least includes as follows. Firstly, a guidewire is pushed through a puncture site along a human lumen to a lesion site. To avoid vascular damage during the pushing process, a head end of a pilot guidewire is generally soft, so that after the guidewire reaches the lesion site, a distal (front) portion of the pilot guidewire cannot be directly used to recanalize an occluded segment at the lesion, and thus it is necessary to insert a catheter and exchange for different types of guidewires through the catheter until the guidewire is capable of passing through the occluded lesion. Secondly, a balloon catheter is pushed along the guidewire, a thrombolytic catheter, or other rotational atherectomy debulking devices to further expand a size of an inner lumen of the occluded segment. The treatment process may further include, but is not limited to, stent implantation for stent-assisted angioplasty. The entire process requires the alternating use of recanalization and expansion devices, resulting in cumbersome operation and long operation time. Moreover, for the first step, due to the occlusive lesion caused by atherosclerosis, the occluded segment is accompanied by a hard fibrous cap and calcified plaques, with a hard texture, resulting in problems such as long guidewire recanalization time, vascular wall injury, and low recanalization success rate. A failure recanalization of the occluded segment is currently one of main causes of failure in endovascular interventional therapy procedures for occlusive lesions. For the second step, since a balloon cannot achieve true debulking, vascular recoil leads to poor dilation effect, and even thrombus detachment and vascular rupture. To solve these problems, debulking devices such as shockwave balloons, thrombolytic catheters, rotational atherectomy catheters, and aspiration catheters have been developed to remove thrombus and calcification, thereby providing a larger effective lumen diameter. When the debulking effect is poor, a stent needs to be implanted to dilate the lumen, but stent implantation stimulates endothelial hyperplasia, resulting in in-stent restenosis and a very high medium-to-long term occlusion rate.

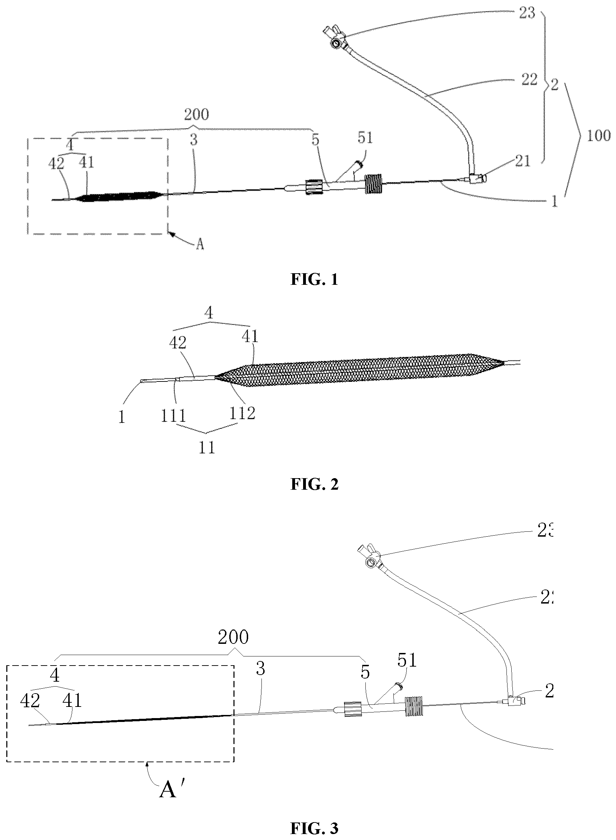

In contrast, a lumen recanalization device is provided in the embodiments. Referring to FIGS. 1 to 4, the lumen recanalization device includes an inner tube assembly 100 and an outer tube assembly 200.

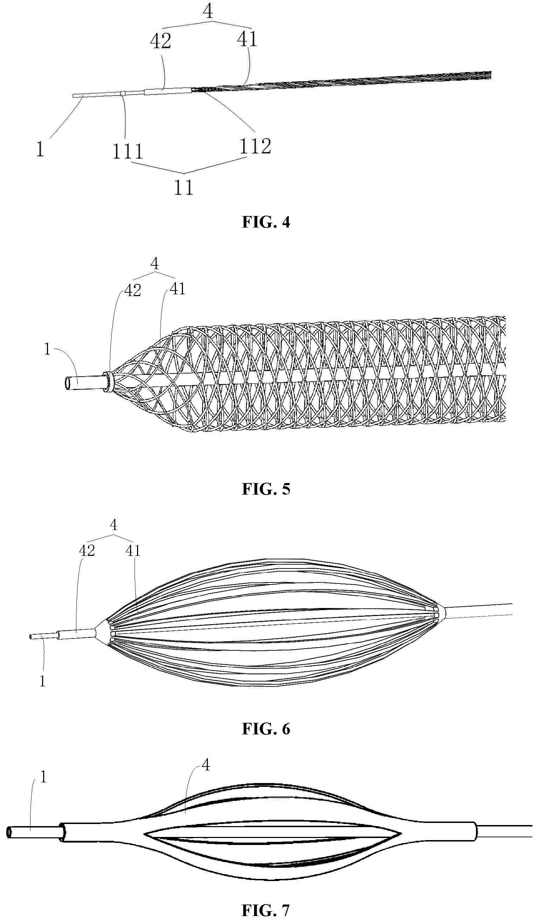

Specifically, the inner tube assembly 100 includes an inner tube 1 and an ultrasonic connector assembly 2, and the ultrasonic connector assembly 2 is connected to a proximal end of the inner tube 1. The outer tube assembly 200 includes an outer tube 3 and at least one expansion-contraction portion 4 connected to a distal end of the outer tube 3. The outer tube 3 and these expansion-contraction portions 4 are all sleeved outside the inner tube 1. Each expansion-contraction portion 4 is configured to have a contracted state where it integrally adheres to or fits closely to an outer wall of the inner tube 1 as shown in FIGS. 3 and 4, and an expanded state where it bulges outward from the inner tube 1 along a radial direction of the inner tube 1 as shown in FIGS. 1 and 2. The outer tube assembly 200 is capable of moving relative to the inner tube 1 to enable the expansion-contraction portion 4 to transition between the two states.

In the embodiments, the inner tube 1 and the outer tube 3 are made of rigid or semi-rigid materials, such as, but not limited to, metal tubes or metal-braided composite tubes.

A usage method and at least achievable functional effects of the lumen recanalization device provided in the embodiments are as follows.

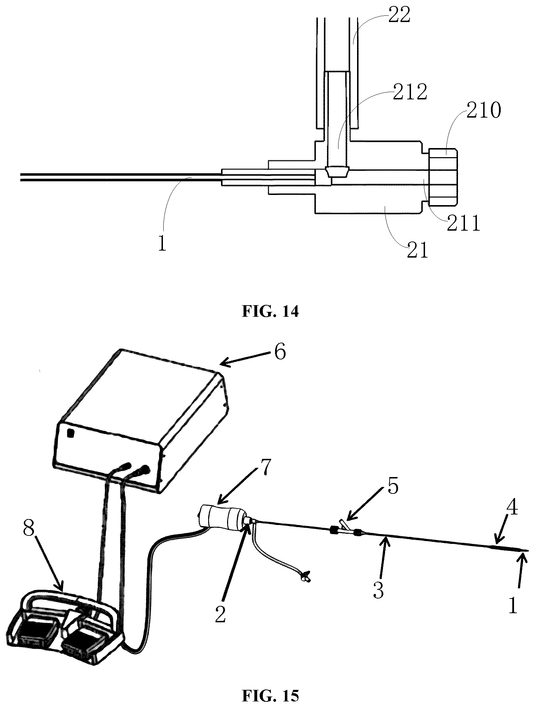

An ultrasonic device is required during use. As generally shown in FIG. 15, the ultrasonic device includes an ultrasonic generator 6 and an ultrasonic transducer 7 connected to each other. Some ultrasonic devices further include a foot-driven pedal 8 shown in FIG. 15. The ultrasonic generator 6 may be driven by the foot-driven pedal 8 to start the ultrasonic device in cooperation. There are also ultrasonic devices with other driving methods, which all belong to the prior art and will not be repeated in the present disclosure.

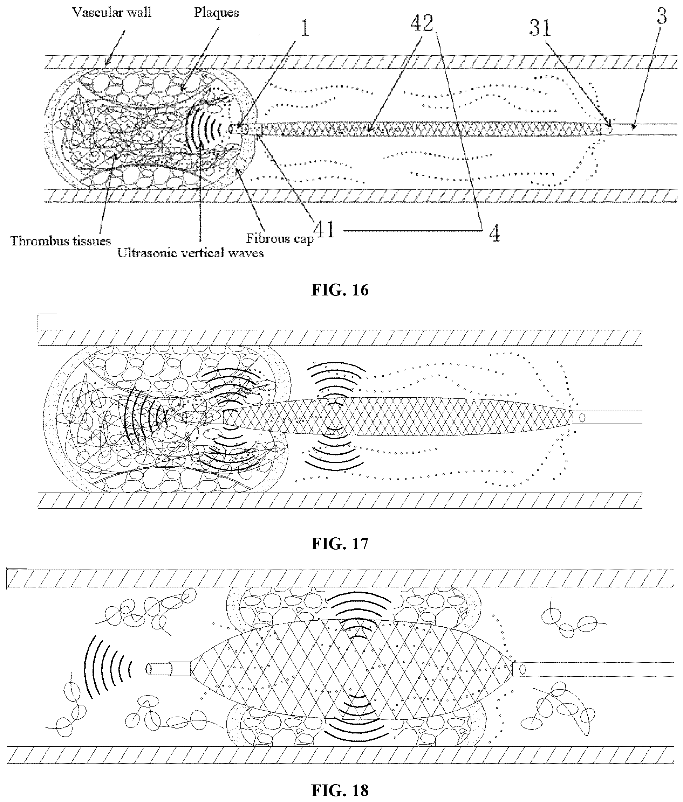

Referring to FIGS. 1 to 4 and FIGS. 15 to 18, the ultrasonic connector assembly 2 at the proximal end of the inner tube 1 is connected to the ultrasonic transducer 7 of the ultrasonic device, and each expansion-contraction portion 4 is ensured to be in the contracted state, and then a distal end of the inner tube 1 of the lumen recanalization device is extended into an occluded site of a lesion.

Then, in a first step, the ultrasonic device is started to recanalize the occluded site with the help of ultrasonic energy. This recanalization method mainly applies a combined mechanism of mechanical vibration and cavitation, and uses ultrasonic energy for lumen recanalization. Mechanical vibrations at an ultrasonic frequency reach a lesion location along the recanalization device. When the device vibrates at a high frequency, the cavitation occurs, and tiny bubbles are generated around the lesion. These tiny bubbles oscillate in alternating periodic vibration waves, and the bubbles are alternately stretched and compressed. At a moment when the bubbles are compressed to collapse, an instantaneous pressure is generated, and this instantaneous pressure jump forms a shock wave. The explosion of countless bubbles is like countless micro-bombs, causing damage to an internal structure of an occluded segment, thereby allowing the device to quickly pass through the lesion location while expanding the lumen. More specifically, the high-frequency and low-amplitude mechanical vibrations generated by ultrasound may cause the distal end of the inner tube 1 to penetrate the hard fibrous cap like a vibrating "hand-held hammer". The high-frequency vibrations of the inner tube 1 generate strong negative and positive pressure cycles in the surrounding fluid. When the distal end of the inner tube 1 retracts, dissolved gases on a negative side of a pressure cycle form cavitation bubbles, which rapidly collapse on a positive side of the pressure cycle. The collapse of cavitation bubbles produces strong mechanical shock waves that erode calcified plaques. Moreover, therapeutic ultrasound for the recanalization of the occluded segment also has the characteristic of selective plaque ablation. Tissues with high collagen and elastin content show strong resistance to ultrasonic damage, while tissues lacking these components are easily destroyed. High-intensity ultrasonic waves disrupt fibrous or calcified plaques without damaging normal arterial vessels. This recanalization method of the occluded segment is more efficient, which is conducive to greatly shortening the recanalization time of the occluded segment during the procedure, and avoiding the problem that the guidewire cannot pass through due to an excessive hardness of the occluded segment, thereby improving the recanalization success rate of the occluded segment.

In a second step, the outer tube assembly 200 is operated to move the outer tube assembly 200 relative to the inner tube 1, so that each expansion-contraction portion 4 transitions from the contracted state shown in FIGS. 3 and 4 to the expanded state shown in FIGS. 1 and 2, thereby expanding a hole formed by recanalization in the first step to further expand a size of an inner lumen of the occluded segment while supporting the inner tube 1 to indirectly improve a supporting force for supporting the guidewire (the guidewire is a conventional pilot guidewire for interventional procedures, not shown in the present disclosure) extended into the inner tube 1. Under an action of this strong supporting force, the guidewire may have a strong directionality and is not easy to bend, thereby further shortening recanalization time of the occluded segment and improving recanalization efficiency of the occluded segment.

In particular, the first step and the second step described above may also be carried out in a reverse order. That is, the outer tube assembly 200 is first operated to move the outer tube assembly 200 relative to the inner tube 1, so that each expansion-contraction portion 4 transitions from the contracted state shown in FIGS. 3 and 4 to the expanded state shown in FIGS. 1 and 2. The expansion-contraction portion 4 in the expanded state supports the inner tube 1. Then, the ultrasonic device is started to recanalize the occluded site with the help of ultrasonic energy. Preferably, the first step and the second step are alternatively performed according to the needs of an operator.

In summary, the lumen recanalization device provided in the embodiments alleviates the problem that the device is difficult to pass through the occluded segment of the lesion in existing endovascular interventional therapy procedures for occlusive lesions, and achieves good effects of shortening the recanalization time of the occluded segment and improving the recanalization efficiency of the occluded segment. Moreover, as analyzed above, surgical instruments are not replaced during the entire process of recanalizing and dilating the occluded segment of the lesion, thereby alleviating the problem of cumbersome operation caused by the alternating use of the recanalization and expansion devices in existing endovascular interventional therapy procedures for occlusive lesions, and alleviating the problem of low long-term patency rate caused by the in-stent restenosis due to the stent implantation required by poor debulking effect of balloon dilation. Compared with the prior art, the lumen recanalization device provided in the embodiments is simpler and more convenient to use, and the surgical process is faster and more convenient, which is conducive to further improving the surgical efficiency, reducing the surgical risk of various complications caused by excessive operation time, and further improving an immediate success rate and medium-to-long effects of a procedure (It should be noted that the effects achievable by the lumen recanalization device provided in the present disclosure are only relative to the lumen recanalization devices provided in the prior art and most patient conditions, and due to numerous influencing factors in an actual surgical treatment, it does not mean that the use of the lumen recanalization device provided in the present disclosure can guarantee the absolute safety and effectiveness of treatment for any patient with a related disease).

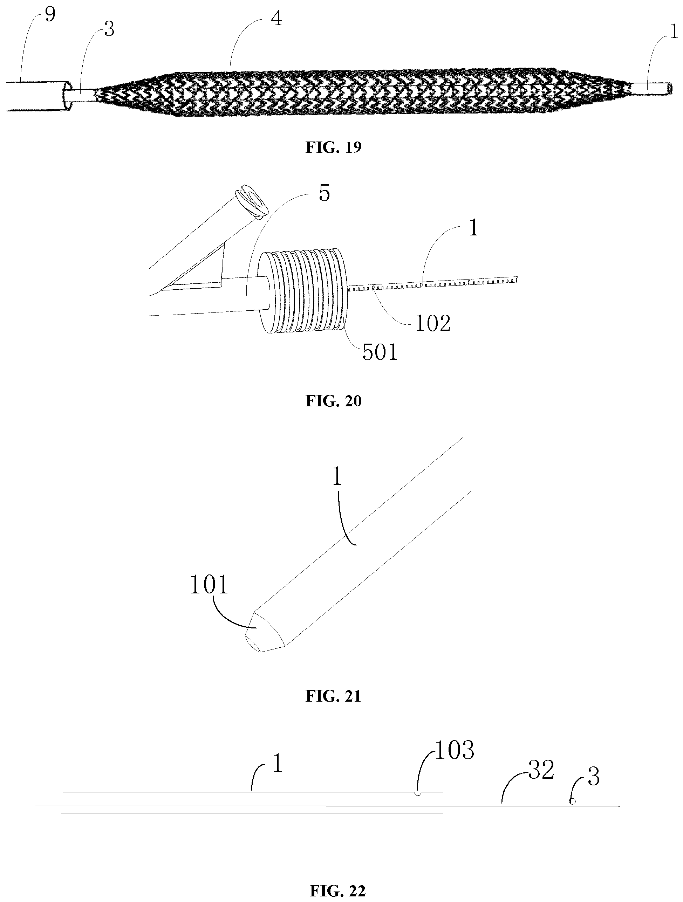

As shown in FIG. 21, in the embodiments, a distal portion of the inner tube 1 is preferably provided with a tip 101, but it is not limited thereto. The tip 101 may be a conical hollow guide head detachably connected (such as clamped or screwed) or fixedly connected or integrally connected to the distal end of the inner tube 1, or a sharp structure formed by gradually thinning a wall thickness of the inner tube 1 at the distal portion in a direction from the proximal end to the distal end of the inner tube 1. Specifically, in the embodiments, the inner tube assembly 100 and the outer tube assembly 200 may further include more tube structures, which are sleeved in cooperation with the inner tube 1 and the outer tube 3 shown in FIGS. 1 to 4 to meet other functional requirements. However, in the most basic structure of the embodiments, the inner tube assembly 100 at least includes the inner tube 1 shown in FIGS. 1 to 4, and the outer tube assembly 200 at least includes the outer tube 3 described above.

The specific structure of the lumen recanalization device provided in the embodiments is described in more detail below.

Specifically, in the embodiments, as shown in FIGS. 1 to 4, the distal end of the inner tube 1 may extend beyond a portion of these expansion-contraction portions 4 most proximate to the distal end of the inner tube 1, or an end face of the distal end of the inner tube 1 may be flush with an end face of a distal end of the expansion-contraction portion 4, or the distal end of the inner tube 1 may be located inside the expansion-contraction portion 4. Preferably, as shown in FIGS. 1 to 4, the distal end of the inner tube 1 extends beyond the portion of these expansion-contraction portions 4 most proximate to the distal end of the inner tube 1, so that during the ultrasonic recanalization process, the distal end of the inner tube 1 is not interfered with by the expansion-contraction portion 4 and is in sufficient contact with the occluded site, thereby ensuring that the distal end of the inner tube 1 quickly recanalizes the occluded segment of the lesion during the ultrasonic recanalization.

Continuing to refer to FIGS. 1 to 4, in the embodiments, an expansion-contraction portion 4 most proximate to the distal end of the inner tube 1 is a distal expansion-contraction portion. In some optional implementations, the expansion-contraction portion(s) 4 only include the distal expansion-contraction portion, that is, there is one and only one expansion-contraction portion 4. In some other optional implementations, there are at least two expansion-contraction portions 4, and in these expansion-contraction portions 4, an expansion-contraction portion 4 farthest from the distal end of the inner tube 1 is a proximal expansion-contraction portion. These expansion-contraction portions 4 are sequentially connected end to end, and the distal end of the outer tube 3 is connected to a proximal end of the proximal expansion-contraction portion; or, the outer tube 3 includes a proximal tube and a plurality of distal tubes, these expansion-contraction portions 4 are spaced from each other, two adjacent expansion-contraction portions 4 are connected through at least one distal tube, and a distal end of the proximal tube is connected to the proximal end of the proximal expansion-contraction portion.

Specific transition methods for the outer tube 3 to move relative to the inner tube 1 to enable the expansion-contraction portion 4 to transition between the expanded state shown in FIGS. 1 and 2 and the contracted state shown in FIGS. 3 and 4 include at least the following two major categories of optional methods.

In a first major category of optional methods, in these expansion-contraction portions 4, the portion most proximate to the distal end of the inner tube 1 is fixed to an outer wall of the distal end of the inner tube 1, or as shown in FIGS. 2 and 4, the outer wall of the distal end of the inner tube 1 is provided with a limiting structure 11, and the portion most proximate to the distal end of the inner tube 1 is limited to the outer wall of the distal end of the inner tube 1 by the limiting structure 11. By rotating the outer tube 3 relative to the inner tube 1 or sliding the outer tube 3 back and forth relative to the inner tube 1, the state transition of the expansion-contraction portion 4 is then controlled.

Specifically, referring to FIGS. 1 to 12, the expansion-contraction portion 4 includes a framework 41, and the framework 41 is in a mesh shape or formed by a plurality of connection wires extending along an axial direction of the inner tube 1 and arranged at intervals.

In an example where the state transition of the expansion-contraction portion 4 is controlled by rotating the outer tube 3 relative to the inner tube 1, it is necessary to fix the portion of these expansion-contraction portion 4 most proximate to the distal end of the inner tube 1 to the outer wall of the distal end of the inner tube 1. A free state of the expansion-contraction portion 4 is the expanded state with a certain resilience. In the contracted state, the expansion-contraction portion 4 may be wound around an outer peripheral wall of the inner tube 1, and rotating relative to the inner tube 1 may release the expansion-contraction portion 4 to make the expansion-contraction portion 4 return to the expanded state.

In an example where the state transition of the expansion-contraction portion 4 is controlled by sliding the outer tube 3 back and forth relative to the inner tube 1, the portion most proximate to the distal end of the inner tube 1 is fixed to the outer wall of the distal end of the inner tube 1, or as shown in FIGS. 2 and 4, the outer wall of the distal end of the inner tube 1 is provided with the limiting structure 11, and the portion most proximate to the distal end of the inner tube 1 is limited to the outer wall of the distal end of the inner tube 1 by the limiting structure 11. The free state of the expansion-contraction portion 4 is the expanded state with a certain resilience. In the contracted state, the outer tube 3 may straighten the expansion-contraction portion 4 toward a proximal direction, and pushing the expansion-contraction portion 4 forward relative to the inner tube 1 may release the expansion-contraction portion 4 to make the expansion-contraction portion 4 return to the expanded state. It is also possible that the free state of the expansion-contraction portion 4 is the contracted state. Since the portion of these expansion-contraction portion 4 most proximate to the distal end of the inner tube 1 is fixed to the outer wall of the distal end of the inner tube 1 or limited to the outer wall of the distal end of the inner tube 1 by the limiting structure 11 provided on the outer wall of the distal end of the inner tube 1, so that when the outer tube 3 is pushed toward a distal direction relative to the inner tube 1, the expansion-contraction portion 4 may be axially compressed, thereby enabling the expansion-contraction portion 4 to transition to the expanded state.

In the above structures, in the expansion-contraction portion 4 connected to the distal end of the outer tube 3, the portion most proximate to the distal end of the inner tube 1 is fixed to the outer wall of the distal end of the inner tube 1 or limited to the outer wall of the distal end of the inner tube 1 by the limiting structure 11, that is, the expansion-contraction portion 4 connected to the distal end of the outer tube 3 is fixed together (in contact) with the distal end of the inner tube 1. Therefore, while the inner tube 1 vibrates ultrasonically, ultrasonic energy of the inner tube 1 may be transmitted to the expansion-contraction portion 4 connected to the distal end of the outer tube 3. In addition, the movement of the outer tube assembly 200 relative to the inner tube 1 may enable each expansion-contraction portion 4 to transition from the contracted state to the expanded state. The expansion-contraction portion 4 in the expanded state may cut thrombus, fit closely to the vascular wall and crush calcified plaques attached to the wall, thereby further shortening the recanalization time of the occluded segment and improving the recanalization efficiency of the occluded segment.

In the above two control forms of the outer tube 3 sliding back and forth relative to the inner tube 1 and rotating relative to the inner tube 1, the control form in which the outer tube 3 is capable of sliding back and forth relative to the inner tube 1 is further preferred to enable the expansion-contraction portion 4 to transition between the expanded state and the contracted state. Moreover, the expansion-contraction portion 4 preferably adopts the above structure in which the free state is the contracted state. This structure may adjust a bulging degree of the expansion-contraction portion 4 in the expanded state by pushing and pulling the outer tube 3 relative to the inner tube 1, so that the expansion-contraction portion 4 adapts to vascular walls or occluded segment holes of various diameters, which is conducive to better transmission of ultrasonic energy from the inner tube 1 to the thrombus and vascular wall through the expansion-contraction portion 4. More specifically, when the outer tube 3 slides toward the distal direction relative to the inner tube 1, the expansion-contraction portion 4 transitions from the contracted state to the expanded state; when the outer tube 3 slides toward the proximal direction relative to the inner tube 1, the expansion-contraction portion 4 transitions from the expanded state to the contracted state.

In a second major category of optional methods, as shown in FIG. 19, the outer tube assembly 200 further includes an outer sheath tube 9. The outer sheath tube 9 is sleeved outside the outer tube 3. The outer sheath tube 9 is capable of sliding back and forth relative to the outer tube 3, so as to retract or release the expansion-contraction portion 4 in a gap between an inner wall of the outer sheath tube 9 and an outer wall of the outer tube 3, thereby controlling the state transition of the expansion-contraction portion 4. Specifically, the free state of the expansion-contraction portion 4 is the expanded state with a certain resilience. In an initial state, the expansion-contraction portion 4 is retracted in the gap between the inner wall of the outer sheath tube 9 and the outer wall of the outer tube 3. By withdrawing the outer sheath tube 9 backward relative to the outer tube 3, the expansion-contraction portion 4 may gradually emerge from a distal end of the outer sheath tube 9. Finally, the expansion-contraction portion 4 is released into the foregoing gap and restores to the expanded state.

The specific structure of the expansion-contraction portion 4 of the embodiments is described in detail below with reference to FIGS. 1 to 12.

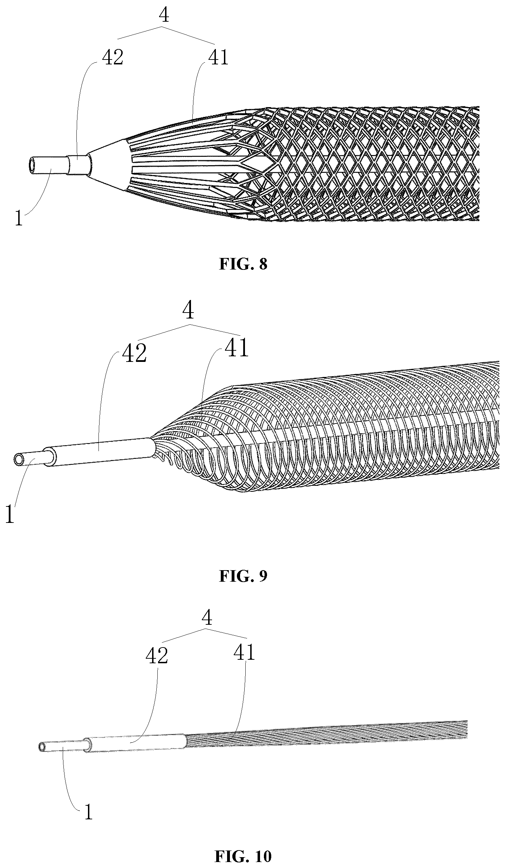

Referring to FIGS. 1 to 12, the expansion-contraction portion 4 includes a framework 41, and the framework 41 may be processed from connection lines of straight, curved, filamentous, strip-shaped, mesh-shaped, spiral-shaped, or other shapes. The connection lines may be made of materials, including, but not limited to, metal materials, other high-performance non-metallic materials, or composite materials, and are preferably made of metal materials such as stainless steel, nitinol, cobalt-based, or titanium-based materials. As shown in FIGS. 5 and 8, the framework 41 is in a mesh shape, or as shown in FIG. 6, the framework 41 is formed by a plurality of connection wires extending along the axial direction of the inner tube 1 and arranged at intervals. The framework 41 may be constrained by proximal ends and distal ends of the connection wires, or as shown in FIG. 7, may be cut from an integral pipe and bulged in a middle.

The expansion-contraction portion 4 most proximate to the distal end of the inner tube 1 is the distal expansion-contraction portion, and the outer tube 3 is capable of rotating relative to the inner tube 1 or sliding back and forth relative to the inner tube 1 to enable the expansion-contraction portion 4 to transition between the expanded state and the contracted state.

As shown in FIGS. 9 and 10, in some optional implementations, a distal end of the framework 41 of the distal expansion-contraction portion is fixedly connected to the outer wall of the distal end of the inner tube 1 by integral connection, adhesion, riveting, welding, or other fixed connection methods.

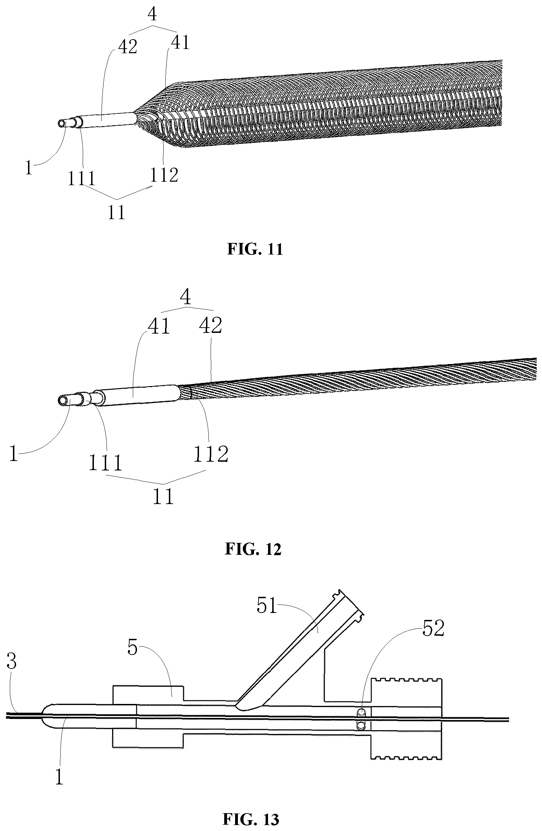

As shown in FIGS. 5, 6, and 8 to 12, in some optional implementations, the distal expansion-contraction portion further includes a constraint fixation component 42 connected to the distal end of the framework 41. The constraint fixation component 42 of the distal expansion-contraction portion may be fixed to the outer wall of the distal end of the inner tube 1, and fixation methods include, but are not limited to, integral connection, adhesion, riveting, welding, or other fixed connections, as well as clamping and other methods; or the constraint fixation component 42 is limited to the outer wall of the distal end of the inner tube 1 by the limiting structure 11. Continuing to refer to FIGS. 5, 6, and 8 to 12, in these optional implementations, the constraint fixation component 42 includes a fixing ring or a fixing tube in structure, and the fixing ring or the fixing tube is sleeved outside the inner tube 1. Moreover, the constraint fixation component 42 may be integrally formed with the framework 41 or separately welded or otherwise fixedly connected to the framework 41. Continuing to refer to FIGS. 11 and 12 in combination with FIGS. 1 to 4, in a case where the outer tube 3 is capable of sliding back and forth relative to the inner tube 1 to enable the expansion-contraction portion 4 to transition between the expanded state and the contracted state, as shown in FIGS. 11 and 12, the limiting structure 11 preferably includes a distal blocking protrusion 111, but it is not limited thereto. The constraint fixation component 42 of the distal expansion-contraction portion is blocked on a proximal side of the distal blocking protrusion 111. When the outer tube 3 is pushed toward the distal direction relative to the inner tube 1, the constraint fixation component 42 of the distal expansion-contraction portion contacts the distal blocking protrusion 111, and the distal blocking protrusion 111 blocks the constraint fixation component 42 of the distal expansion-contraction portion from continuing to slide toward the distal direction relative to the inner tube 1. Thus, the expansion-contraction portion 4 is axially compressed and radially bulged to the expanded state. When the outer tube 3 slides toward the proximal direction relative to the inner tube 1, the expansion-contraction portion 4 returns to its original state. To further limit a movement range of each expansion-contraction portion 4 and improve an operation accuracy, preferably, as shown in FIGS. 2, 4, 11, and 12, the limiting structure 11 further includes a proximal blocking protrusion 112, but it is not limited thereto. The proximal blocking protrusion 112 is located on a proximal side of the constraint fixation component 42 of the distal expansion-contraction portion. The proximal blocking protrusion 112 and the distal blocking protrusion 111 jointly limit the constraint fixation component 42.

In addition, in a preferred implementation of the embodiments, the expansion-contraction portion 4 further includes a membrane connected to the framework 41, and the membrane is made of a flexible material such as polyurethane (PU), nylon, nylon elastomer (Pebax), polyethylene terephthalate (PET), polytetrafluoroethylene (PTFE), polyvinyl fluoride (PVF); polyvinyl formal, fluorinated ethylene propylene (FEP), silicone rubber, polyimide (PI). The membrane is configured to be coated with thrombolytic drugs or anti-endothelial hyperplasia drugs such as paclitaxel or rapamycin. In some optional implementations, the membrane is a bare membrane without drug coating. In some other optional implementations, the membrane is pre-coated with drugs. The membrane may be sutured or connected to the framework 41 by coating, adhesion, welding, dip coating, spraying and other methods, such as, but not limited to the following methods. A film-forming solution is prepared in advance. The solution may be made of a biocompatible material in which a high-molecular polymer is soluble in an organic solvent. The framework 41 is immersed in the solution and removed. Due to small gaps between the connection lines of the framework 41, especially the mesh-shaped framework, a surface tension of the solution may form a liquid film suspended in the gaps of the framework 41. After the solution evaporates, materials such as the high-molecular polymer are left to form a thin film and adhere to a surface of the framework 41, thereby forming the membrane. A thickness of the membrane may be controlled by a concentration of the solution or a number of coating times. Alternatively, two ends of a hollow balloon processed from a polymeric material by an extrusion blow molding process are welded to the framework 41 by laser welding, so as to obtain the membrane. Drugs may be attached to a surface of the membrane by drug coating, spraying, impregnation, and other methods to achieve drug loading on the membrane. The purpose of providing the membrane in this preferred implementation is mainly to increase a contact area between the expansion-contraction portion 4 in the expanded state and the vascular wall of the lesion, so that granular drugs or drug layers adhere to the surface of the membrane. In the expanded state, the expansion-contraction portion 4 expands the framework 41 and the membrane, and the surface of the membrane fits closely to the lesion site. Drugs are delivered by directly adhering to the lesion, and in cooperation with ultrasonic vibrations, the drugs are detached from the surface of the membrane, and drug dissolution is accelerated under an action of the ultrasonic vibrations, so that the drugs are absorbed by diseased tissues for local treatment of the lesion site. Especially for anti-endothelial hyperplasia drugs such as rapamycin and paclitaxel, which are toxic and should not be used in large quantities, if no membrane is provided, applying only a drug layer to the framework 41 results in an insufficient contact area with the lesion site, an inadequate drug administration, and poor adhesion of the drug to the surface of the vascular wall. Therefore, compared with applying the drug layer to the framework 41 or directly injecting the drug into the blood vessel for treatment, the method of contacting the lesion after the membrane is expanded not only improves a drug utilization rate but also reduces toxicity.

For ease of operation, referring to FIGS. 1 to 4 and 13, in the optional implementations of the embodiments, preferably, the outer tube assembly 200 further includes a sliding handle 5, but it is not limited thereto. The sliding handle 5 is connected to a proximal end of the outer tube 3 and sleeved outside the inner tube 1. The sliding handle 5 is configured to drive the outer tube 3 to slide back and forth relative to the inner tube 1. Preferably, referring to FIG. 20, the sliding handle 5 is further provided with a sliding position mark 501, but it is not limited thereto. An outer wall of the proximal end of the inner tube 1 is provided with scale lines 102 arranged along the axial direction of the inner tube 1. The sliding position mark 501 and the scale line 102 cooperate with each other to display a distance that the outer tube 3 slides back and forth relative to the inner tube 1. The sliding position mark 501 may include, but is not limited to, a marking ring, a marking protrusion, or a marking line provided on an outer peripheral wall of the sliding handle 5. When the sliding position mark 501 is at a distance from an end face of a proximal (rear) end of the sliding handle 5, the entire sliding handle 5 or at least a portion of the sliding handle 5 corresponding to a proximal (rear) side of the sliding position mark 501 is made of a transparent or translucent material for the operator to observe, or an edge of the end face of the proximal end of the sliding handle 5 may be used as the sliding position mark 501.

To avoid blood leakage during operation, in some preferred implementations of the embodiments, in addition to the outer tube 3 and the expansion-contraction portion(s) 4, the outer tube assembly 200 further includes a sealing device 52. The sealing device 52 is configured to seal a tube gap between an inner wall of the proximal end of the outer tube 3 and the outer wall of the inner tube 1 along the radial direction. Specifically, a specific arrangement of the sealing device 52 includes, but is not limited to, the following. Whether the sliding handle 5 is provided or not, the sealing device 52 is arranged in the tube gap between the inner wall of the proximal end of the outer tube 3 and the outer wall of the inner tube 1, thereby directly sealing the gap between the inner wall of the proximal end of the outer tube 3 and the outer wall of the inner tube 1 along the radial direction. Alternatively, as shown in FIG. 13, the sliding handle 5 is provided, and the sealing device 52 is arranged between an inner wall of the sliding handle 5 and the outer wall of the inner tube 1 to seal a gap between the inner wall of the sliding handle 5 and the outer wall of the inner tube 1, thereby indirectly sealing the gap between the inner wall of the proximal end of the outer tube 3 and the outer wall of the inner tube 1 along the radial direction. In addition, as shown in FIG. 13, the sealing device 52 may adopt a sealing ring structure or a structure such as an annular rear cover (not shown) provided at the proximal end (rear end) of the outer tube 3 or at the proximal end (rear end) of the sliding handle 5, but it is not limited thereto.

In addition, studies show that with the assistance of ultrasonic energy, thrombolytic agents such as streptokinase and urokinase enter an interior of thrombus, which may significantly accelerate a speed of thrombus dissolution. In contrast, in some preferred implementations of the embodiments, as shown in FIG. 13, in a case where the sliding handle 5 is provided, a handle liquid passage hole 51 is additionally provided on the sliding handle 5. The handle liquid passage hole 51 is communicated with the gap between the outer tube 3 and the inner tube 1, and the handle liquid passage hole 51 is located on a distal side of the sealing device 52. Alternatively, in some other preferred implementations of the embodiments, whether the sliding handle 5 is provided or not, as shown in FIG. 22, an outer tube liquid passage hole 32 penetrating a tube wall of the outer tube 3 is provided on a wall of the proximal end of the outer tube 3. In a case where both the outer tube liquid passage hole 32 and the sealing device 52 are provided, the outer tube liquid passage hole 32 is located on the distal side of the sealing device 52. In these preferred implementations, by injecting thrombolytic drugs into the handle liquid passage hole 51 or the outer tube liquid passage hole 32, ultrasonic energy may accelerate a penetration speed of the thrombolytic drugs into an interior of the thrombus, which greatly improves a dissolution efficiency of the thrombus, thereby achieving effective debulking and lumen dilation, reducing a ratio of stent implantation, and improving short term and medium-to-long term treatment effects. The injected thrombolytic drugs may flow out from a distal orifice of the outer tube 3; or as shown in FIGS. 14 to 16, a liquid spraying hole 31 may be provided on a side wall of the distal end of the outer tube 3, so that the thrombolytic drugs are capable of bing sprayed from a side of the outer tube 3 to better contact the vascular wall. In the embodiments, the handle liquid passage hole 51 or the outer tube liquid passage hole 32 may also be used to inject other diagnostic and therapeutic liquids such as contrast agents. In the embodiments, by providing the handle liquid passage hole 51 or the outer tube liquid passage hole 32, the lumen recanalization device also has a function of a thrombolytic catheter, which avoids a step of separately introducing the thrombolytic catheter to inject a thrombolytic liquid, thereby further improving the surgical efficiency.

In addition, in some optional implementations of the embodiments, a liquid may be injected into the inner tube 1 through the proximal end of the inner tube 1 to cool the inner tube 1 and the outer tube 3, thereby ensuring that a temperature within a surgical environment does not become too high. Specifically, the structure may be that, as shown in FIG. 22, an inner tube liquid passage hole 103 penetrating a tube wall of the inner tube 1 is provided on a wall of the proximal end of the inner tube 1, and the liquid is injected through the inner tube liquid passage hole 103; or the structure may be that, referring to FIGS. 1 to 4 and FIGS. 14 and 15, the ultrasonic connector assembly 2 includes an ultrasonic connector 21, and a proximal end of the ultrasonic connector 21 is provided with a connection portion 210 configured to be connected to the ultrasonic transducer 7. An inner tube connection hole 211 and a connector liquid passage hole 212 are provided on the ultrasonic connector 21. The proximal end of the inner tube 1 is connected to a distal end of the inner tube connection hole 211, a lumen of the inner tube 1 is communicated with the inner tube connection hole 211, and the connector liquid passage hole 212 is communicated with the inner tube connection hole 211. The liquid is injected through the connector liquid passage hole 212 to cool the inner tube 1 and the outer tube 3. Certainly, a negative pressure device may also be connected through the connector liquid passage hole 212 to form a negative pressure inside the inner tube 1, so as to aspirate debris generated during a recanalization of a thrombus site or during a thrombolytic process. In the structure where the liquid is injected through the connector liquid passage hole 212 to cool the inner tube 1 and the outer tube 3, it is further preferred that the ultrasonic connector assembly 2 further includes a hose 22 and a check valve assembly 23. An end of the hose 22 is connected to the connector liquid passage hole 212. The check valve assembly 23, which only allows the liquid to flow from the hose 22 into the inner tube connection hole 211 through the connector liquid passage hole 212, is connected to a portion of the hose 22 away from the connector liquid passage hole 212. The liquid is injected into the connector liquid passage hole 212 through the hose 22 and the check valve assembly 23 during an ultrasound operation.

It should be particularly noted that in the above a plurality of optional or preferred implementations of the embodiments, the outer tube liquid passage hole 32, the handle liquid passage hole 51, the inner tube liquid passage hole 103, and the above connector liquid passage hole 212 are not limited to the type of injected liquid. For example, the outer tube liquid passage hole 32 and the handle liquid passage hole 51 may also be used to inject an ultrasonic cooling liquid, and the inner tube liquid passage hole 103 and the connector liquid passage hole 212 may also be used to inject other diagnostic and therapeutic liquids such as thrombolytic drugs or contrast agents. In addition, the outer tube liquid passage hole 32, the handle liquid passage hole 51, the inner tube liquid passage hole 103, and the above connector liquid passage hole 212 may be selectively set simultaneously, alternatively, or in any combination.

A lumen recanalization system is further provided in the embodiments. As shown in FIG. 15, the lumen recanalization system includes an ultrasonic generator 6, an ultrasonic transducer 7, and the lumen recanalization device provided in any of the optional implementations of the first embodiment. The ultrasonic transducer 7 is connected to the ultrasonic generator 6, the ultrasonic connector assembly 2 is connected to the ultrasonic transducer 7, and the ultrasonic generator 6 may be driven by a foot-driven pedal 8 shown in FIG. 15, but it is not limited thereto.

In addition, a lumen recanalization method is further provided in the embodiments of the present disclosure. The lumen recanalization method uses the lumen recanalization system provided in the second aspect described above. A distal end of the lumen recanalization device is fed into an occluded site in a vascular lumen of a patient, and the ultrasonic generator 6 is started to drive the inner tube 1 to vibrate. While the inner tube 1 vibrates ultrasonically, ultrasonic energy of the inner tube 1 may be transmitted to the expansion-contraction portion(s) 4 connected to the distal end of the outer tube 2, so that the expansion-contraction portion 4 in the expanded state cuts thrombus.

Since the lumen recanalization method provided in the embodiments of the present disclosure uses the above lumen recanalization system, which in turn includes the above lumen recanalization device, the lumen recanalization method and the lumen recanalization system provided in the embodiments of the present disclosure are capable of achieving all the beneficial effects achievable by the above lumen recanalization device.

Finally, the following should be noted.
1. The lumen recanalization device, the lumen recanalization system, and the lumen recanalization method provided in the embodiments of the present disclosure are not only limited to a surgical scenario of vascular recanalization. Lumens to which the lumen recanalization device and the lumen recanalization system may be applied further include other human lumens such as a ureter and a bile duct.
2. The embodiments in the description are described in a progressive manner, and each embodiment focuses on difference(s) from other embodiments. For the same and similar parts among the various embodiments, reference may be made to each other. The above embodiments in the description are only used to describe the technical solutions of the present disclosure, rather than to limit them. Although the present disclosure is described in detail with reference to the foregoing embodiments, those of ordinary skill in the art should understand that they can still modify the technical solutions described in the foregoing embodiments, or perform equivalent replacements for some or all of the technical features in the foregoing embodiments. These modifications or replacements do not make the essence of the corresponding technical solutions depart from the scope of the technical solutions of the various embodiments of the present disclosure.

### INDUSTRIAL PRACTICALITY

In summary, the embodiments of the present disclosure provide the lumen recanalization device, the lumen recanalization system, and the lumen recanalization method, which at least alleviate the problems in existing endovascular interventional therapy procedures for occlusive lesions, such as the difficulty of the device in passing through the occluded segment of the lesion, the cumbersome operation of the device, and the low long-term patency rate caused by the in-stent restenosis due to stent implantation required by poor debulking effect of balloon dilation, thereby improving the immediate success rate and the medium-to-long term effects of the procedure. The present disclosure has industrial practicability.

## Claims

1. A lumen recanalization device, **characterized by** comprising an inner tube assembly (100) and an outer tube assembly (200), wherein
the inner tube assembly (100) comprises an inner tube (1) and an ultrasonic connector assembly (2), and the ultrasonic connector assembly (2) is connected to a proximal end of the inner tube (1); and
the outer tube assembly (200) comprises an outer tube (3) and at least one expansion-contraction portion (4) connected to a distal end of the outer tube (3), wherein
both the outer tube (3) and the at least one expansion-contraction portion (4) are sleeved outside the inner tube (1); and
the expansion-contraction portion (4) is configured to have a contracted state where it integrally adheres to or fits closely to an outer wall of the inner tube (1), and an expanded state where it bulges outward from the inner tube (1) along a radial direction of the inner tube (1), and the outer tube assembly (200) is capable of moving relative to the inner tube (1) to enable the expansion-contraction portion (4) to transition between the expanded state and the contracted state.

2. The lumen recanalization device according to claim 1, wherein a distal end of the inner tube (1) extends beyond a portion of the at least one expansion-contraction portion (4) most proximate to the distal end of the inner tube (1).

3. The lumen recanalization device according to claim 2, wherein a distal portion of the inner tube (1) is provided with a tip (101).

4. The lumen recanalization device according to any one of claims 1 to 3, wherein an expansion-contraction portion (4) most proximate to a distal end of the inner tube (1) is a distal expansion-contraction portion, wherein:
the expansion-contraction portion (4) only comprises the distal expansion-contraction portion;
or,
at least two expansion-contraction portions (4) are provided, and an expansion-contraction portion (4) farthest from the distal end of the inner tube (1) is a proximal expansion-contraction portion, wherein the at least two expansion-contraction portions (4) are sequentially connected end to end, and the distal end of the outer tube (3) is connected to a proximal end of the proximal expansion-contraction portion; or the outer tube (3) comprises a proximal tube and a plurality of distal tubes, the at least two expansion-contraction portions (4) are spaced from each other, two adjacent expansion-contraction portions (4) are connected through at least one distal tube, and a distal end of the proximal tube is connected to the proximal end of the proximal expansion-contraction portion.

5. The lumen recanalization device according to any one of claims 1 to 4, wherein a portion of the at least one expansion-contraction portion (4) most proximate to a distal end of the inner tube (1) is fixed to an outer wall of a distal end of the inner tube (1).

6. The lumen recanalization device according to any one of claims 1 to 4, wherein an outer wall of a distal end of the inner tube (1) is provided with a limiting structure (11), and a portion of the at least one expansion-contraction portion (4) most proximate to the distal end of the inner tube (1) is limited to the outer wall of the distal end of the inner tube (1) by the limiting structure (11).

7. The lumen recanalization device according to claim 6, wherein an expansion-contraction portion (4) most proximate to the distal end of the inner tube (1) is a distal expansion-contraction portion, and a distal end of the distal expansion-contraction portion is further connected to a constraint fixation component (42).

8. The lumen recanalization device according to claim 7, wherein the constraint fixation component (42) comprises a fixing ring or a fixing tube, and the fixing ring or the fixing tube is sleeved outside the inner tube (1).

9. The lumen recanalization device according to claim 7 or 8, wherein the outer tube (3) is capable of sliding back and forth relative to the inner tube (1) to enable the expansion-contraction portion (4) to transition between the expanded state and the contracted state; and the limiting structure (11) comprises a distal blocking protrusion (111) provided on an outer wall of the distal end of the inner tube (1), and the constraint fixation component (42) of the distal expansion-contraction portion is blocked on a proximal side of the distal blocking protrusion (111).

10. The lumen recanalization device according to claim 9, wherein the limiting structure (11) further comprises a proximal blocking protrusion (112), and the proximal blocking protrusion (112) is located on a proximal side of the constraint fixation component (42) of the distal expansion-contraction portion.

11. The lumen recanalization device according to any one of claims 1 to 10, wherein the outer tube assembly (200) further comprises an outer sheath tube (9), the outer sheath tube (9) is sleeved outside the outer tube (3), and the outer sheath tube (9) is capable of sliding back and forth relative to the outer tube (3), so as to retract or release the expansion-contraction portion (4) in a gap between an inner wall of the outer sheath tube (9) and an outer wall of the outer tube (3).

12. The lumen recanalization device according to any one of claims 1 to 11, wherein
the expansion-contraction portion (4) comprises a framework (41), and the framework (41) is in a mesh shape or is formed by a plurality of connection wires extending along an axial direction of the inner tube (1) and arranged at intervals.

13. The lumen recanalization device according to claim 12, wherein the expansion-contraction portion (4) further comprises a membrane connected to the framework (41), and the membrane is made of a flexible material.

14. The lumen recanalization device according to any one of claims 1 to 13, wherein the outer tube assembly (200) further comprises a sealing device (52), and the sealing device (52) is configured to seal a gap between an inner wall of a proximal end of the outer tube (3) and the outer wall of the inner tube (1) along a radial direction.

15. The lumen recanalization device according to claim 14, wherein the outer tube assembly (200) further comprises a sliding handle (5), the sliding handle (5) is connected to the proximal end of the outer tube (3) and sleeved outside the inner tube (1), and the sliding handle (5) is configured to drive the outer tube (3) to slide back and forth relative to the inner tube (1).

16. The lumen recanalization device according to claim 15, wherein the sealing device (52) is arranged between an inner wall of the sliding handle (5) and the outer wall of the inner tube (1) to seal a gap between the inner wall of the sliding handle (5) and the outer wall of the inner tube (1) along the radial direction, thereby indirectly sealing the gap between the inner wall of the proximal end of the outer tube (3) and the outer wall of the inner tube (1) along the radial direction.

17. The lumen recanalization device according to claim 16, wherein a handle liquid passage hole (51) is provided on the sliding handle (5), the handle liquid passage hole (51) is communicated with a gap between the outer tube (3) and the inner tube (1); and the handle liquid passage hole (51) is located on a distal side of the sealing device (52).

18. The lumen recanalization device according to claim 14 or 15, wherein the sealing device (52) is arranged in a tube gap between the inner wall of the proximal end of the outer tube (3) and the outer wall of the inner tube (1), thereby directly sealing a gap between the inner wall of the proximal end of the outer tube (3) and the outer wall of the inner tube (1) along the radial direction.

19. The lumen recanalization device according to any one of claims 15 to 17, wherein a sliding position mark (501) is provided on the sliding handle (5), a scale line (102) arranged along the axial direction of the inner tube (1) is provided on an outer wall of the proximal end of the inner tube (1), and the sliding position mark (501) and a scale line (102) cooperate with each other to display a distance that the outer tube (3) slides back and forth relative to the inner tube (1).

20. The lumen recanalization device according to any one of claims 1 to 19, wherein an outer tube liquid passage hole (32) penetrating a tube wall of the outer tube (3) is provided on a wall of a proximal end of the outer tube (3).

21. The lumen recanalization device according to any one of claims 1 to 20, wherein an inner tube liquid passage hole (103) penetrating a tube wall of the inner tube (1) is provided on a wall of the proximal end of the inner tube (1).

22. The lumen recanalization device according to any one of claims 1 to 21, wherein
the ultrasonic connector assembly (2) comprises an ultrasonic connector (21); and
a proximal end of the ultrasonic connector (21) is provided with a connection portion (210) configured to be connected to an ultrasonic transducer (7); the ultrasonic connector (21) is provided with an inner tube connection hole (211) and a connector liquid passage hole (212), the proximal end of the inner tube (1) is connected to a distal end of the inner tube connection hole (211) and a lumen of the inner tube (1) is communicated with the inner tube connection hole (211), and the connector liquid passage hole (212) is communicated with the inner tube connection hole (211).

23. The lumen recanalization device according to claim 22, wherein the ultrasonic connector assembly (2) further comprises a hose (22) and a check valve assembly (23); an end of the hose (22) is connected to the connector liquid passage hole (212), and the check valve assembly (23) that only allows a liquid to flow from the hose (22) into the inner tube connection hole (211) through the connector liquid passage hole (212) is connected to a portion of the hose (22) away from the connector liquid passage hole (212).

24. A lumen recanalization system, **characterized by** comprising an ultrasonic generator (6), an ultrasonic transducer (7), and the lumen recanalization device according to any one of claims 1 to 23, wherein the ultrasonic transducer (7) is connected to the ultrasonic generator (6), and the ultrasonic connector assembly is connected to the ultrasonic transducer (7).

25. A lumen recanalization method, **characterized by** comprising using the lumen recanalization system according to claim 24 to feed a distal end of the lumen recanalization device into an occluded site in a vascular lumen of a patient, and starting the ultrasonic generator (6) to drive the inner tube (1) to vibrate, wherein while the inner tube (1) vibrates ultrasonically, ultrasonic energy of the inner tube (1) is capable of being transmitted to the expansion-contraction portion (4) connected to the distal end of the outer tube (3), so that the expansion-contraction portion (4) in the expanded state cuts thrombus.
